# EUROPÄISCHE PATENTANMELDUNG

(11) **EP 0 855 183 A2**
(43) Veröffentlichungstag der Anmeldung: **29.07.1998**
(21) Anmeldenummer: 98100766.9
(22) Anmeldetag: 17.01.1998
(51) Int. Cl.: A61K 31/495, A61K 9/16, A61K 9/20

(54) **Herstellung geschmackskaschierter Zubereitungen antibakteriell wirksamer Chinolonderivaten**

(30) Priorität: 24.01.1997 DE 19702443
(71) Anmelder: HOECHST AKTIENGESELLSCHAFT, 65929 Frankfurt am Main (DE)
(72) Erfinder: Ahrens, Gerhard, Dr., 65835 Liederbach (DE); Mentrup, Edgar, Dr., 60437 Frankfurt (DE); Maas, Jochen, Dr., 64390 Erzhausen (DE); Radau, Manfred, Dr., 65779 Kelkheim (DE)

(57) **Zusammenfassung**

Die vorliegende Erfindung betrifft ein Verfahren zur Herstellung geschmackskaschierter oraler pharmazeutischer Zubereitungen antibakteriell wirksamer Chinolonderivate sowie durch dieses Verfahren herstellbare Zubereitungen.

## Beschreibung

Die vorliegende Erfindung betrifft ein Verfahren zur Herstellung geschmackskaschierter oraler pharmazeutischer Zubereitungen antibakteriell wirksamer Chinolonderivate.

Es ist bekannt, daß antibakteriell wirksame Hemmstoffe der bakteriellen Gyrase aus der Substanzgruppe der Chinolone sich durch einen stark bitteren Eigengeschmack auszeichnen. Dieser Eigengeschmack muß zur Erzielung ausreichender Patientenakzeptanz bei Verabreichung oraler Zubereitungen der Substanzen kaschiert sein. In der Regel geschieht dies durch Filmlackierung der Tabletten. Zur Therapie mit antibakteriell wirksamen Chinolonderivaten sind Einzeldosen von 100 mg bis 1000 mg des Artneistoffs erforderlich. Die Einnahme entsprechend großvolumiger fester Arzneiformen durch Schlucken bereitet älteren sowie auch schluckbehinderten Patienten und Kindern große Schwierigkeiten, so daß Bedarf an einer geschmacklich akzeptablen oralen Arzneiform besteht, die nicht als Ganzes in fester Form geschluckt werden muß, sondern die entweder in Flüssigkeit suspendiert oder durch Zerkauen eingenommen werden kann.

Die technologisch einfachste Art und Weise der Überdeckung eines unangenehmen Geschmacks stellt die Zugabe geeigneter Aromen zu einer Lösung des Arzneistoffs dar. Im Falle der antibakteriell wirksamen Chinolonderivate ist diese Maßnahme allerdings unzureichend, so daß weitere Schritte zur Kaschierung oder Neutralisation des bitteren Geschmacks erforderlich sind.
Die bisher bekannten Methoden zielen darauf ab, die Auflösung der überwiegend gut löslichen antibakteriell wirksamen Chinolonderivate in dem verwendeten Lösungsmittel und im Speichel zu verhindern oder wenigstens zu verringern.

In EP-A-0551 820 geschieht dies durch Mikroverkapselung des Anhydrates der Basisform dieser Substanzen. Als Hüllmaterialien werden wasserunlösliche neutrale Methyl- und/oder Ethyl-Ester-Verbindungen oder quartäre Ammoniumverbindungen der Polymethacrylsäure oder Mischungen daraus oder Ethylcellulose verwendet.

In Biol. Pharm. Bull. (1993), 16 (2), 172-7 wird eine Mikroverkapselung feiner Granulate aus Sparfloxacin und niedrig substituierter Hydroxypropylcellulose unter Verwendung von Ethylcellulose und Hydroxypropylmethylcellulose als Hüllmaterialien beschrieben.

Ein ähnliches Verfahren zur Mikroverkapselung von Partikeln aus Pyridoncarbonsäure - antibakteriellen Mitteln (speziell Enoxacin) und in Wasser quellbaren Mitteln mit Mischungen aus Ethylcellulose und wasserlöslichen Polymeren wird in EP-A- 409 254 beschrieben.

Bei diesen Verfahren zur Mikroverkapselung werden die Hüllmaterialien durch Aufsprühen ihrer Lösungen aufgebracht. Nachteilig ist hierbei, daß aufwendig konstruierte Apparaturen erforderlich sind und daß der nutzbaren Partikelgröße der zu umhüllenden Granulate eine praktische Untergrenze gesetzt ist - zum einen bedingt durch den mit abnehmender Partikelgröße überproportional ansteigenden Lackbedarf und zum anderen durch die mit abnehmender Partikelgröße zunehmende Kohäsivität, die die Möglichkeiten zum Umhüllen mittels Aufsprühen auf Grund zunehmender Agglomerationstendenzen technisch einschränkt.

So nennt die EP-A- 0551 820 als bevorzugten Bereich für die erhaltenen Mikrokapseln 100 - 500 µm. Partikeln von 500 µm, als Suspension appliziert, können aber durchaus ein unangenehm sandiges Gefühl im Mund des Patienten erzeugen. Zudem ist der Zeitbedarf für die Umhüllung feiner Partikeln durch Aufsprühen hoch.
JP 63 150220 beschreibt ein Verfahren, das auch ein Produkt ähnlich einer Mikroverkapselung ergeben dürfte. Pulverförmiger Arzneistoff (z.B. Enoxacin) wird mit pulverförmigem Hüllmaterial gemischt, z.B. mit wachsartigen Substanzen, wie Paraffin, Stearinsäure, Bienenwachs etc. oder mit polymeren, zur Filmbildung fähigen Substanzen, wie Methacrylsäure Copolymer, Ethylcellulose, Polystyrol etc.. Die Mischung wird mit organischen Lösemitteln versetzt, um die Hüllmaterialien aufzulösen. Nach Entfernen des Lösemittels wird wieder ein Pulver erhalten. Nachteilig an diesem Verfahren ist die Verwendung von brennbaren, gesundheitsschädlichen, oder ökologisch bedenklichen Lösungsmitteln, die aufwendig zurückzugewinnen bzw. zu entsorgen sind. Es verbleibt zudem eine Restmenge im Produkt.

Allen bisher beschriebenen Verfahren zur Umhüllung von Wirkstoffpartikeln oder Granulaten antimikrobiell wirksamer Chinolonderivate ist die Verwendung flüssiger Träger als Medium zur Auflösung oder Dispergierung der Hüllmaterialien gemeinsam.

Der Erfindung lag die Aufgabe zugrunde, ein Verfahren zur Herstellung oral applizierbarer geschmackskaschierter Arzneizubereitungen mit schneller Wirkstofffreisetzung antibakteriell wirksamer Chinolonderivate zur Verfügung zu stellen, das die geschilderten Nachteile der literaturbekannten Verfahren nicht aufweist und das zudem eine Verwendung flüssiger Träger als Medium zur Auflösung oder Dispergierung der Hüllmaterialien vermeidet.
Unter schneller Wirkstofffreisetzung wird eine mindestens 80 %ige Freisetzung einer Einzeldosis nach 30 Minuten in 900 ml 0,1 N HCl verstanden.

Überraschend wurde nun gefunden, daß eine Geschmackskaschierung auch ohne Verwendung flüssiger Träger möglich ist, wenn man antimikrobiell wirksame Chinolonderivate mit höheren Fettsäuren und gegebenenfalls weiteren Zuschlagstoffen mischt, die Mischung erhitzt und nach dem Erkalten zu einem Pulver oder Granulat zerkleinert. Das Produkt weist selbst nach einer Pulverisierung noch eine ausreichende Geschmackskaschierung auf. Die Erhitzung der Mischungen kann nach herkömmlichen Verfahren vorgenommen werden. Aus verfahrenstechnischer Sicht bevorzugt ist die Verwendung eines Heizmischers hoher Drehzahl (wie z.B. Henschel Fluidmischer FM4 oder FM10), in dem die Mischungen durch Friktionswärme aufgeheizt werden und der im gleichen Aggregat die Möglichkeit des Zerkleinerns nach dem Erkalten bietet. Unter hoher Drehzahl wird eine Drehzahl von mindestens 3000 UpM verstanden. Die Mischungen werden auf 30°C bis 140°C, bevorzugt 40°C bis 120°C, besonders bevorzugt auf 50°C bis 85°C erhitzt.

Die Erfindung betrifft weiterhin Zubereitungen, die nach diesem Verfahren herstellbar sind.

Unter höheren Fettsäuren werden Fettsäuren mit mindestens 10 C-Atomen verstanden.
Geeignete höhere Fettsäuren sind solche mit 10 bis 22 Kohlenstoffatomen, wobei Fettsäuren mit 12 bis 18 Kohlenstoffatomen für das beschriebene Verfahren besonders geeignet sind. Auch Gemische von Fettsäuren sind vorteilhaft einsetzbar. So ist z.B. handelsübliche Stearinsäure, die ein Gemisch aus etwa gleichen Mengen Stearinsäure und Palmitinsäure darstellt, gut einsetzbar. Ein geeigneter weiterer Zuschlagstoff ist z.B. hochdisperses Siliciumdioxid.

Die antibakteriell wirksamen Chinolinderivate besitzen als essentielles Strukturmerkmal die 1-Ethyl-4-oxo-pyridin-3-carbonsäure (siehe Auterhoff, Knabe, Höltje, Lehrbuch der Pharmazeutischen Chemie, 13. Auflage,m 1994, Seite 788). Bevorzugt sind Levofloxacin, Ofloxacin, Ciprofloxacin, Norfloxacin, Sparfloxacin oder Enoxacin. Besonders bevorzugt sind Levofloxacin und Ofloxacin.

Das Gewichtsverhältnis von Chinolonderivat(en) zu Fettsäure(en) beträgt vorzugsweise von 1:0,3 bis 1:4, besonders bevorzugt: 1:1 bis 1:2.

Die erfindungsgemäß hitzebehandelten und anschließend zerkleinerten Zubereitungen aus antimikrobiell wirksamem Chinolonderivat und höherer Fettsäure können zu Arzneimitteln weiterverarbeitet werden, wie z.B. zu Sachets, Kautabletten oder Trinktabletten.

### 1. Trinktabletten

Eine Trinktablette ist eine Arzneiform, die dazu bestimmt ist, in einem Glas Wasser innerhalb von höchstens 3 Minuten unter Rühren zu zerfallen und eine Suspension zu bilden, die ein Sieb von 0,715 mm Maschenweite passieren kann.
Trinktabletten bieten sich als Arzneiform für hoch zu dosierende Arzneistoffe an, die als konventionelle Tablette auf Grund ihres großen Volumens schwer schluckbar wären. Die besondere Applikationsweise der Trinktablette in Suspensions- oder Lösungsform erfordert eine hinreichende Geschmackskaschierung schlecht schmeckender Arzneistoffe.
Trinktabletten können mit Hilfsstoffen, die auch für konventionelle Tabletten Verwendung finden, formuliert werden. Typischerweise kommen noch Aromen, Süßstoffe und evtl. Farbstoffe hinzu. Brauchbare Herstelltechnologien sind Feuchtgranulation, Trockengranulation und Direkttablettierung.

### 2. Sachets

Sachets sind Beutel, die meistens eine Einzeldosis eines Arzneimittels als Pulver oder Granulat enthalten. Zur Applikation wird der Inhalt eines Beutels in einem Glas Wasser suspendiert oder aufgelöst. Geeignete Zuschlagstoffe sind neben Zuckern und Zuckeraustauschstoffen viskositätserhöhende Hilfsstoffe, Fließregulierungsmittel, Aromastoffe, Farbstoffe und evtl. Puffersubstanzen.

### 3. Kautabletten

Kautabletten sind Tabletten, die vor dem Schlucken zerbissen werden. Gründe für den Einsatz dieser Arzneiform sind im Falle der hochdosierten antibakteriell wirksamen Chinolonderivate ebenfalls Probleme, die bestimmte Patientengruppen mit der Schluckbarkeit konventioneller Tabletten, Filmtabletten, Kapseln oder Dragees haben. Kautabletten enthalten neben anderen üblichen Tablettierhilfsstoffen meistens überwiegend Zucker oder Zuckeraustauschstoffe und auch Aromen.

Die Wirkstofffreisetzung wird in einer USP paddle Apparatur gemessen, wie sie in USP23, Seite 1792, Abschnitt 711, Figur 2 beschrieben ist.

### Ausführungsbeispiele

### Beispiel 1

zeigt die Herstellung einer geschmackskaschierten Grundmischung von Levofloxacin - Hemihydrat.

| | |
|---|---|
| Levofloxacin - Hemihydrat | 256,23 mg |
| Stearinsäure | 211,00 mg |

Levofloxacin - Hemihydrat und Stearinsäure werden in einem Heizmischer bei einer Drehzahl von 1000 UpM über 3 Minuten gemischt. Man erhöht anschließend die Drehzahl auf 5500 UpM und mischt weiter bis die Temperatur des Mischgutes 75°C erreicht und das Gut eine granulatartige Struktur angenommen hat. Man kühlt durch Mantelkühlung auf Raumtemperatur ab und zerkleinert das Gut anschließend durch weiteres Mischen über 4 x 0,5 Minuten bei 5500 UpM unter weiterer Kühlung. Es wird ein geschmacksneutrales Pulver erhalten.

Die Wirkstofffreisetzungsbestimmungen in einer USP paddle - Apparatur ergaben folgende Ergebnisse:

| | Freisetzungsmedium | |
|---|---|---|
| | 900 ml Wasser | 900 ml 0,1 N Salzsäure |
| nach 5 Minuten | 34,2 % | 91,2 % |
| nach 15 Minuten | 47,1 % | 97,9 % |
| nach 30 Minuten | 55,7 % | 97,4 % |

### Beispiel 2

zeigt die Weiterverarbeitung der geschmackskaschierten Grundmischung aus Beispiel 1 zu einer Trinktablette

| | |
|---|---|
| Levofloxacin - Hemihydrat/Stearinsäuremischung | 467,23 mg |
| Maisstärke | 243,77 mg |
| Mikrokristalline Cellulose | 200,00 mg |
| Crospovidone mikronisiert | 50,00 mg |
| Polyvinylpyrrolidon | 5,00 mg |

Levofloxacin - Hemihydrat/Stearinsäuremischung, Maisstärke, Mikrokristalline Cellulose, Crospovidone mikronisiert und Polyvinylpyrrolidon werden gemischt, mit Wasser granuliert, getrocknet, über ein Sieb der Maschenweite 1,2 mm gepreßt und mit einer Exzentertablettiermaschine tablettiert.

In der Suspension der Trinktablette in 100 ml Leitungswasser von Raumtemperatur ist der bittere Eigengeschmack des Wirkstoffs weitgehend kaschiert.

Die Wirkstofffreisetzungsbestimmungen in einer USP paddle - Apparatur ergaben folgende Ergebnisse:

| | Freisetzungsmedium | |
|---|---|---|
| | 900 ml Wasser | 900 ml 0,1 N Salzsäure |
| nach 5 Minuten | 32,2 % | 96,4 % |
| nach 15 Minuten | 47,4 % | 100 % |
| nach 30 Minuten | 58,9 % | 100 % |

### Beispiel 3

zeigt die Herstellung einer Levofloxacin - Hemihydrat Trinktablette mit Palmitinsäure als geschmackskaschierender höherer Fettsäure, die nach dem erfindungsgemäßen Verfahren hergestellt wurde:

| | |
|---|---|
| Levofloxacin - Hemihydrat | 512,46 mg |
| Palmitinsäure | 422,00 mg |
| Mikrokristalline Cellulose | 457,54 mg |
| Crospovidone | 150,00 mg |
| Himbeer - Aroma | 30,00 mg |
| Aspartam | 60,00 mg |

Levofloxacin - Hemihydrat und Palmitinsäure werden, wie in Beispiel 1 beschrieben, zu einer geschmackskaschierten Grundmischung verarbeitet.
Mikrokristalline Cellulose, Crospovidone, Himbeeraroma und Aspartam werden zugemischt und die Gesamtmischung kompaktiert und über ein 1,0 mm Sieb trocken granuliert. Das Trockengranulat wird zu Trinktabletten verarbeitet.

Die Wirkstofffreisetzungsbestimmungen in einer USP paddle - Apparatur ergaben folgende Ergebnisse:

| | Freisetzungsmedium | |
|---|---|---|
| | 900 ml Wasser | 900 ml 0,1 N Salzsäure |
| nach 5 Minuten | 43,5 % | 100 % |
| nach 15 Minuten | 58,9 % | 100 % |
| nach 30 Minuten | 73,9 % | 100 % |

### Beispiel 4

zeigt die Herstellung einer Norfloxacin Trinktablette nach dem erfindungsgemäßen Verfahren mit Stearinsäure als geschmackskaschierender höherer Fettsäure. Norfloxacin ist ein Beispiel für einen Arzneistoff mit geringerer Löslichkeit als Levofloxacin.

| | |
|---|---|
| Norfloxacin | 500,00 mg |
| Stearinsäure | 600,00 mg |
| Maisstärke | 711,46 mg |
| Mikrokristalline Cellulose | 638,32 mg |
| Crospovidone | 146,22 mg |
| Hochdisp. Siliciumdioxid | 14,00 mg |
| Pfirsich-Aroma | 30,00 mg |
| Aspartam | 40,00 mg |
| Acesulfam K | 20,00 mg |

Norfloxacin und Stearinsäure werden, analog Beispiel 1, zu einer geschmackskaschierten Grundmischung verarbeitet. Maisstärke, Mikrokristalline Cellulose, Crospovidone, Hochdisperses Siliciumdioxid, Pfirsich-Aroma, Aspartam und Acesulfam K werden der Grundmischung zugemischt. Die Gesamtmischung wird mit einer Exzentertablettiermaschine tablettiert.

Die Wirkstofffreisetzungsbestimmungen in einer USP paddle - Apparatur ergaben folgende Ergebnisse:

| | Freisetzungsmedium | |
|---|---|---|
| | 900 ml Wasser | 900 ml 0,1 N Salzsäure |
| nach 5 Minuten | 7,6 % | 94,9 % |
| nach 15 Minuten | 16,5 % | 99,5% |
| nach 30 Minuten | 24 % | 100 % |

### Beispiel 5

zeigt die Herstellung einer Ofloxacin Trinktablette nach dem erfindungsgemäßen Verfahren mit Stearinsäure als geschmackskaschierender höherer Fettsäure.

| | |
|---|---|
| Ofloxacin | 500,00 mg |
| Stearinsäure | 600,00 mg |
| Maisstärke | 711,46 mg |
| Mikrokristalline Cellulose | 638,32 mg |
| Crospovidone | 146,22 mg |
| Hochdisp. Siliciumdioxid | 14,00 mg |
| Pfirsich-Aroma | 30,00 mg |
| Aspartam | 40,00 mg |
| Acesulfam K | 20,00 mg |

Ofloxacin und Stearinsäure werden analog Beispiel 1 zu einer geschmackskaschierten Grundmischung verarbeitet. Maisstärke, Mikrokristalline Cellulose, Crospovidone, Hochdisperses Siliciumdioxid, Pfirsich-Aroma, Aspartam und Acesulfam K werden der Grundmischung zugemischt, die Gesamtmischung kompaktiert, über ein 1,0 mm Sieb granuliert und mit einer Exzentertablettiermaschine tablettiert.

Die Wirkstofffreisetzungsbestimmungen in einer USP paddle - Apparatur ergaben folgende Ergebnisse:

| | Freisetzungsmedium | |
|---|---|---|
| | 900 ml Wasser | 900 ml 0,1 N Salzsäure |
| nach 5 Minuten | 6,8 % | 87,3 % |
| nach 15 Minuten | 14,4 % | 100 % |
| nach 30 Minuten | 20,6 % | 100 % |

### Beispiele 6, 7, 8 und 9

zeigen die Herstellung von Levofloxacin - Hemihydrat Trinktabletten unter Verwendung verschiedener wachsartiger Substanzen als geschmackskaschierenden Agenzien.

| Beispiel | 9 | 10 | 11 | 12 |
|---|---|---|---|---|
| Levofloxacin -Hemihydrat | 512,45 mg | 512,45 mg | 512,45 mg | 512,45 mg |
| Stearinsäure | 422,00 mg | --- | --- | --- |
| Stearylalkohol | --- | 422,00 mg | --- | --- |
| Hydriertes Rizinusöl | --- | --- | 422,00 mg | --- |
| Glycerolmonostearat | --- | --- | --- | 422,00 mg |
| Polyvidone 25000 | 10,00 mg | 10,00 mg | 10,00 mg | 10,00 mg |
| Maisstärke | 487,55 mg | 487,55 mg | 487,55 mg | 487,55 mg |
| Mikrokristalline Cellulose | 400,00 mg | 400,00 mg | 400,00 mg | 400,00 mg |
| Crospovidone mikronisiert | 100,00 mg | 100,00 mg | 100,00 mg | 100,00 mg |

Levofloxacin - Hemihydrat und Stearinsäure bzw. Stearylalkohol bzw. Hydriertes Rizinusöl bzw. Glycerolmonostearat werden in einer Reibschale verrieben und in einem Trockenschrank über 1 Stunde auf 80 °C erhitzt. Man läßt abkühlen, pulverisiert die Mischung und mischt mit Polyvidone 25000, Maisstärke, Mikrokristalline Cellulose und Crospovidone mikronisiert. Man granuliert die Mischung mit Wasser, trocknet, passiert durch ein Sieb der Maschenweite 1 mm und verpreßt das Granulat zu Tabletten.

Die Wirkstofffreisetzungsbestimmungen in einer USP paddle - Apparatur ergaben folgende Ergebnisse:

| Beispiel | 9 | 10 | 11 | 12 |
|---|---|---|---|---|
| | | | | |

| Freisetzungsmedium Wasser | | | | |
|---|---|---|---|---|
| nach 5 Minuten | 33,5 % | 64,3 % | 51,9 % | 61,6 % |
| nach 15 Minuten | 46,4 % | 81,7 % | 76,1 % | 74,4 % |
| nach 30 Minuten | 63,4 % | 90,6 % | 89,3 % | 81,5 % |

| Freisetzungsmedium 0,1 N Salzsäure | | | | |
|---|---|---|---|---|
| nach 5 Minuten | 95,3 % | 91,6 % | 81,2 % | 85,1 % |
| nach 15 Minuten | 100 % | 99,4 % | 94,0 % | 93,8 % |
| nach 30 Minuten | 100 % | 100% | 100 % | 98,6 % |

Nach Suspendierung der Trinktabletten aus Beispiel 9 bis 12 in je 100 ml Leitungswasser und Geschmacksbeurteilung ergab sich nur für die Trinktabletten aus Beispiel 9 eine hinreichende Geschmackskaschierung.

### Vergleichsbeispiel 1

zeigt die Herstellung einer Levofloxacin - Hemihydrat Trinktablette mit Palmitinsäure als höherer Fettsäure, die nicht nach dem erfindungsgemäßen Verfahren hergestellt wurde.

| | |
|---|---|
| Levofloxacin - Hemihydrat | 512,46 mg |
| Palmitinsäure | 422,00 mg |
| Mikrokristalline Cellulose | 807,54 mg |
| Crospovidone | 150,00 mg |
| Himbeer - Aroma | 30,00 mg |
| Aspartam | 60,00 mg |

Levofloxacin - Hemihydrat, Palmitinsäure, Mikrokristalline Cellulose, Crospovidone, Himbeer-Aroma und Aspartame werden gemischt, kompaktiert und über ein 1,0 mm Sieb trocken granuliert. Das Trockengranulat wird zu Trinktabletten verarbeitet.

Die Wirkstofffreisetzungsbestimmungen in einer USP paddle - Apparatur ergaben folgende Ergebnisse:

| | Freisetzungsmedium | |
|---|---|---|
| | 900 ml Wasser | 900 ml 0,1 N Salzsäure |
| nach 5 Minuten | 89,9 % | 98 % |
| nach 15 Minuten | 100 % | 100 % |
| nach 30 Minuten | 100 % | 100 % |

Der bittere Eigengeschmack des Arzneistoffs ist nach Suspendierung der Trinktablette in 100 ml Wasser weit stärker feststellbar als bei der Trinktablette aus Beispiel 3.

### Vergleichsbeispiel 2

zeigt die Herstellung einer Norfloxacin Trinktablette, die nicht nach dem erfindungsgemäßen Verfahren und auch ohne Verwendung einer höheren Fettsäure hergestellt wurde.

| | |
|---|---|
| Norfloxacin | 500,00 mg |
| Maisstärke | 711,46 mg |
| Mikrokristalline Cellulose | 638,32 mg |
| Crospovidone | 146,22 mg |
| Hochdisp. Siliciumdioxid | 14,00 mg |
| Pfirsich-Aroma | 30,00 mg |
| Aspartam | 40,00 mg |
| Acesulfam K | 20,00 mg |

Norfloxacin, Maisstärke, Mikrokristalline Cellulose, Crospovidone, Hochdisperses Siliciumdioxid, Pfirsich-Arome, Aspartam und Acesulfam K werden gemischt und zu Tabletten verpreßt.

Die Wirkstofffreisetzungsbestimmungen in einer USP paddle - Apparatur ergaben folgende Ergebnisse:

| | Freisetzungsmedium | |
|---|---|---|
| | 900 ml Wasser | 900 ml 0,1 N Salzsäure |
| nach 5 Minuten | 40,1 % | 98,2 % |
| nach 15 Minuten | 62,2 % | 100 % |
| nach 30 Minuten | 73,2 % | 100 % |

Nach Suspendierung der Trinktabletten aus Beispiel 4 und Vergleichsbeispiel 2 in je 100 ml Leitungswasser ergaben sich folgende Ergebnisse der Geschmacksprüfung: Die Zubereitung aus Beispiel 4 mit nach dem erfindungsgemäßen Verfahren geschmackskaschiertem Wirkstoff wies keinen bitteren Geschmack mehr auf, während die nicht erfindungsgemäße Zubereitung nach Vergleichseispiel 2 den bitteren Eigengeschmack des Wirkstoffs deutlich erkennen ließ.

### Vergleichsbeispiel 3

zeigt die Herstellung einer Ofloxacin Trinktablette, die nicht nach dem erfindungsgemäßen Verfahren und auch ohne Verwendung einer höheren Fettsäure hergestellt wurde.

| | |
|---|---|
| Ofloxacin | 500,00 mg |
| Maisstärke | 711,46 mg |
| Mikrokristalline Cellulose | 638,32 mg |
| Crospovidone | 146,22 mg |
| Hochdisp. Siliciumdioxid | 14,00 mg |
| Pfirsich-Aroma | 30,00 mg |
| Aspartam | 40,00 mg |
| Acesulfam K | 20,00 mg |

Ofloxacin, Maisstärke, Crospovidone, Hochdisperses Siliciumdioxid, Pfirsich-Aroma, Aspartam und Acesulfam K werden gemischt und zu Tabletten verpreßt.

Die Wirkstofffreisetzungsbestimmungen in einer USP paddle - Apparatur ergaben folgende Ergebnisse:

| | Freisetzungsmedium | |
|---|---|---|
| | 900 ml Wasser | 900 ml 0,1 N Salzsäure |
| nach 5 Minuten | 89,9 % | 95,4 % |
| nach 15 Minuten | 100 % | 97,4 % |
| nach 30 Minuten | 100 % | 100 % |

Nach Suspendierung der Trinktabletten aus Beispiel 5 und Vergleichsbeispiel 3 in je 100 ml Leitungswasser ergaben sich folgende Ergebnisse der Geschmacksprüfung: Die Zubereitung aus Beispiel 5 mit nach dem erfindungsgemäßen Verfahren geschmackskaschiertem Wirkstoff wies keinen bitteren Geschmack mehr auf, während die nicht erfindungsgemäße Zubereitung nach Vergleichsbeispiel 3 den unangenehm bitteren Eigengeschmack des Wirkstoffs deutlich erkennen ließ.

## Patentansprüche

1. Verfahren zur Herstellung oral applizierbarer geschmackskaschierter Arzneizubereitungen mit schneller Wirkstofffreisetzung antibakteriell wirksamer Chinolonderivate, dadurch gekennzeichnet, daß man das Chinolonderivat mit mindestens einer höheren Fettsäure vermischt und entweder gleichzeitig oder nachfolgend durch Erhitzen auf eine Temperatur von 30°C bis 140°C bringt.

2. Verfahren gemäß Anspruch 1, dadurch gekennzeichnet, daß die Temperatur 40°C bis 120°C beträgt.

3. Verfahren gemäß Anspruch 1 oder 2, dadurch gekennzeichnet, daß die Temperatur 65°C bis 85°C beträgt.

4. Verfahren gemäß einem oder mehreren der Ansprüche 1 bis 3, dadurch gekennzeichnet, daß ein Heizmischer hoher Drehzahl zum Erhitzen verwendet wird.

5. Verfahren gemäß einem oder mehreren der Ansprüche 1 bis 4, dadurch gekennzeichnet, daß die Temperaturerhöhung ausschließlich durch die im Mischer entstehende Fiktionswärme erzielt wird.

6. Verfahren gemäß einem oder mehreren der Ansprüche 1 bis 5, dadurch gekennzeichnet, daß das Gewichtsverhältnis von Chinolonderivat(en) zu Fettsäure(n) 1 : 0,3 bis 1 : 4 beträgt.

7. Verfahren gemäß einem oder mehreren der Ansprüche 1 bis 6, dadurch gekennzeichnet, daß man Fettsäuren mit mindestens 10 Kohlenstoffatomen einsetzt.

8. Verfahren gemäß einem oder mehreren der Ansprüche 1 bis 7, dadurch gekennzeichnet, daß man Fettsäuren mit 10 bis 18 Kohlenstoffatomen einsetzt.

9. Verfahren gemäß einem oder mehreren der Ansprüche 1 bis 8, dadurch gekennzeichnet, daß als antibakteriell wirksame Chinolonderivate Chinolonderivate wie Levofloxacin, Ofloxacin, Ciprofloxacin, Norfloxacin, Sparfloxacin oder Enoxacin eingesetzt werden.

10. Verfahren gemäß einem oder mehreren der Ansprüche 1 bis 9, dadurch gekennzeichnet, daß als antibakteriell wirksame Chinolonderivate Levofloxacin oder Ofloxacin eingesetzt werden.

11. Verfahren gemäß einem oder mehreren der Ansprüche 1 bis 10, dadurch gekennzeichnet, daß man die Mischung aus antibakteriell wirksamem Chinolonderivat, höheren Fettsäuren und gegebenenfalls weiteren Zuschlagstoffen zu Arzneiformen, die einer Geschmackskaschierung des Wirkstoffes bedürfen, wie Trinktabletten, Kautabletten, Sachets usw. weiterverarbeitet.

12. Zubereitungen, herstellbar nach dem Verfahren entsprechend den Ansprüchen 1 bis 11.

13. Zubereitungen gemäß Anspruch 12 zur Herstellung eines Arzneimittels zur Behandlung von bakteriellen Infektionen.
